# EUROPEAN PATENT SPECIFICATION

(11) **EP 4 126 203 B1**
(45) Date of publication and mention of the grant of the patent: **04.09.2024**
(21) Application number: 21712182.1
(22) Date of filing: 19.03.2021
(51) Int. Cl.: A61N 1/362, A61N 1/365, A61N 1/375, A61N 1/39

(54) **CARDIAC RHYTHM MANAGEMENT SYSTEM**
SYSTEM ZUR HERZRHYTHMUSVERWALTUNG
SYSTÈME DE GESTION DU RYTHME CARDIAQUE

(30) Priority: 23.03.2020 US 202062993083 P; 08.06.2020 EP 20178714
(43) Date of publication of application: 08.02.2023
(73) Proprietor: BIOTRONIK SE & Co. KG, 12359 Berlin (DE)
(72) Inventor: DÖRR, Thomas, 12437 Berlin (DE); MUESSIG, Dirk, West Linn, Oregon 97068 (US)
(74) Representative: Biotronik Corporate Services SE
(86) International application number: PCT/EP2021/057080
(87) International publication number: WO 2021/191074

(56) References cited:
- WO-A1-2015/164168
- US-A1- 2014 330 326
- US-A1- 2015 142 069
- US-A1- 2016 008 615

## Description

The present invention is generally directed to a cardiac rhythm management system (CRMS) that can be used for electric stimulation therapy of cardiac arrhythmia. Such a cardiac rhythm management system comprises at least one first implantable stimulation device, for example an implantable leadless pacemaker (II,P), and at least one second implantable stimulation device, for example a subcutaneous implantable cardioverter defibrillator (S-ICD) or an ILP, wherein the at least one first implantable stimulation device comprises a first detection unit adapted to detect a patient's cardiac rhythm and a first processor adapted to analyze the detected patient's cardiac rhythm and to deliver signals for a first antitachycardia pacing therapy, wherein the at least one second implantable stimulation device comprises a second detection unit adapted to detect the patient's cardiac rhythm and a second processor adapted to analyze the detected patient's cardiac rhythm and to deliver signals for shock therapy or a second antitachycardia pacing therapy.

Implantable stimulation devices such as implantable cardiac pacemakers or ILPs are well known medical devices that allow stimulation of the heart of a patient. In general, those medical devices are battery operated and a stimulation component is directly implanted into the heart's ventricle or atrium. Implantable cardiac pacemakers have at least an elongated stimulation lead which reaches from the device housing into a heart chamber where it is anchored. ILPs are miniaturized pacing devices which are entirely implanted into the heart chamber.

Implantable stimulation devices with a defibrillation function are known in the art, as for instance implantable cardioverter-defibrillators (ICD) or subcutaneous implantable cardioverter-defibrillators (SICD). Such devices typically comprise of a device housing and at least one elongated stimulation lead which extends from the housing. The housing of an ICD is typically implanted in a skin pocket below the clavicle, wherein the stimulation lead reaches into the ventricle of the heart where it is fixed. The housing and stimulation lead of an SICD are implanted under the skin (i.e. subcutaneously), in a way that a shock vector that runs through the cardiac ventricles is created between the stimulation electrode(s) of the lead and the SICD housing.

The medical device is chosen according to the patient's cardiac condition, i.e. the required cardiac therapy.

Implantable pacemakers or ILPs are used for patients who suffer from a bradycardia, that is if a heart that beats too slow to fulfil the physiological needs of the patient. The implantable pacemaker or ILP applies electrical stimulation to the heart in order to generate a physiologically appropriate heartrate.

ICDs are used for patients who suffer from ventricular tachycardia and fibrillations. The ICD is able to apply antitachycardia pacing (ATP) therapy (i.e. pacing the heart with a faster stimulation rate than the tachycardia rate) to terminate a tachycardia, or a shock therapy (i.e. high energetic electric shock which is applied to the ventricles to terminate the tachycardia to bring back the heart to a physiological rhythm) if the tachycardia persists after ATP attempts.

SICDs are configured to deliver a shock therapy, but no pacing therapy or ATP therapy. That is due to the distance between stimulation lead and the cardiac chambers, so that a low energetic stimulation pulse could not be delivered effectively to a cardiac pacing site.

An ILP may deliver pacing therapy and ATP, but no shock therapy. Due to the highly restricted device size, it has a small battery capacity and lack of space for charging capacitors required for providing a shock therapy.

Moreover, implantable leads pose a risk to the patient and can therefore be a problem. The lead is an elongated insulated electrode wire which reaches from the device housing into the venous system of the heart where it is anchored in the ventricle. It undergoes different forces and movements with every beat of the heart, which can result in lead dislodgement, insulation failures and lead breach. That problem does not occur with SICDs and ILPs, because these devices have no intracardiac elongated lead. Especially for patients who have no adequate vascular access or are at high risk for infection, no elongated leads can be implanted inside the heart.

However, there are circumstances in which a patient suffers from various cardiac arrhythmias that require different cardiac therapies. In such cases, a CRMS may be implanted comprising at least two medical devices or units.

Furthermore, there exist cardiac arrhythmias for which different therapies are suitable and one treatment is more favorable, e.g. more comfortable, for the patient. Further, some therapies may cause another arrhythmia, so that an additional therapy is required in order to stop this arrhythmia. In practice, ventricular tachycardia, for example, may be treated using ATP therapy or shock therapy, wherein shock therapy is often uncomfortable for patients as the shocks are emitted unexpectedly and may be painful. In addition, shock therapies cause a considerable decrease in the longevity of the battery. Nevertheless, shock therapy is inevitable if a ventricular tachycardia leads to ventricular fibrillation as ATP therapy is not suitable to treat fibrillations.

For instance, a patient who has a contraindication for intracardiac elongated leads and who suffers from ventricular tachycardia requires pacing therapy, ATP and shock therapy. In such case, a CRMS may be implanted comprising at least a first implantable stimulation device and a second implantable stimulation device, wherein the first implantable stimulation device may be an ILP, and the second device an SICD.

According to another example, if a patient who has a contraindication for intracardiac elongated leads and who requires pacing therapy and/or ATP, in the ventricle (or at the HIS bundle) and in the atrium, a CRMS may be implanted comprising at least a first implantable stimulation device and a second implantable stimulation device, wherein the first implantable stimulation device may be a first ILP, and the second device a second ILP.

Cardiac rhythm management systems comprising multiple treatment therapies are, for example, provided by a combination of S-ICD and ILP as disclosed in the prior art documents US 2019/0160285 A1 and US 10,265,534 B2. The coordination of such systems is obligatory in order to provide proper treatment as the therapies may be ineffective if they are applied simultaneously. WO2016007660 discloses a medical device system for delivering electrical stimulation therapy to heart of patient has leadless cardiac pacemaker that modifies defibrillation shock therapy of medical device, after pacemaker determines occurrence of cardiac arrhythmia.

Therefore, the system described in document US 2019/0160285 A1 discloses the use of multiple medical devices that allow the delivery of stimulation therapy for cardiac arrhythmia, wherein a communication between the medical devices of the system is mentioned in order to provide the required coordination. Document US 10,265,534 B2 discloses a further communication method for a system with a leadless first implantable stimulation device and another implanted device comprising the exchange of communication messages.

However, an external or internal communication within a CRMS, i.e. between one implanted medical device and an external device or between two implanted medical devices, has some disadvantages. These disadvantages are mainly due to the fact that the communication requires communication interfaces, which in turn cause increased energy consumption. In addition, the communication interfaces are vulnerable for cyber-attacks. Furthermore, an undisturbed communication channel is required in order to provide a reliably functioning of a CRMS.

Therefore, it is an object of the present invention to provide a cardiac rhythm management system, which benefits from the combined use of a first implantable stimulation device and a second implantable stimulation device providing combined therapy and which is synchronized without using any separate communication channel. Further, it is an object of the present invention to provide a reliable functioning CRMS, which is user-friendly.

This problem is solved by a cardiac rhythm management system with the features of claim 1 as well as with a cardiac rhythm management method for a cardiac rhythm management system with the features of claim 11.

In particular, a cardiac rhythm management system according to the present invention is disclosed comprising at least one first implantable stimulation device, for example an ILP, and at least one second implantable stimulation device, for example a subcutaneous implantable cardioverter defibrillator or an ILP. Both medical devices, i.e. pacing and second implantable stimulation devices, may be implanted in the chest cavity. More precise, the first implantable stimulation device may be implanted in one heart ventricle or atrium and the second implantable stimulation device may be implanted outside the heart. Further, the at least one first implantable stimulation device comprises a first detection unit, e.g. comprising one or more electrodes adapted to be implanted into the heart's tissue, adapted to detect a patient's cardiac rhythm and a first processor adapted to analyze the detected patient's cardiac rhythm and to deliver signals for a first antitachycardia pacing (ATP) therapy. The first implantable stimulation device with the first detection unit and the first processor may be formed as an integral structure.

Furthermore, the at least one second implantable stimulation device comprises a second detection unit adapted to detect the patient's cardiac rhythm and a second processor adapted to analyze the detected patient's cardiac rhythm and to deliver signals for shock therapy or a second antitachycardia pacing (ATP) therapy.

In case that the second implantable stimulation device is a S-ICD, the second detection unit may comprise at least one detection electrode to detect the patient's cardiac rhythm and the second processor may be electrically connected to at least one shock coil and/or a pulse generator via an electrode lead. The electrode lead may be fixed to the pulse generator or may be connected via a connector pin. Further, the S-ICD may be implanted completely subcutaneous, wherein the components are positioned around the heart but not within the heart. The shock coil and a housing of the S-ICD are accommodated such that an electric shock wave produced by the shock coil is transmitted via at least one heart ventricle to the electrically conducting housing of the S-ICD. As there are no components of the S-ICD directly inserted into the heart, the advantages of an S-ICD are consequently a less irritation of the heart, a lower risk of infection and less mechanical stress of the components. The S-ICD may be placed according to anatomical guidance and does not require any imaging systems such as an X-ray imaging system. The electrode lead of the S-ICD may be inserted parallel to the sternum and the housing with the pulse generator and the second processor may be placed at the left chest wall.

According to this embodiment, the first processor of the first implantable stimulation device is adapted to allow delivery of signals for ATP therapy only if the analysis of the patient's cardiac rhythm within a pre-defined preceding time period A reveals a pre-defined tachycardia criterion A' and an absence of a shock therapy, and/or the second processor of the second implantable stimulation device is adapted to allow delivery of signals for shock therapy or a second ATP therapy only if the analysis of the patient's cardiac rhythm within a pre-defined preceding time period B reveals a pre-defined tachycardia criterion B' and an absence of a first antitachycardia pacing therapy provided by the at least one first implantable stimulation device.

Thus, an ATP therapy triggered by the signals produced by the first processor is only allowed to be delivered by the first implantable stimulation device if the above conditions are satisfied. The ATP therapy is triggered by the respective signals of the first processor and provided by a respective first signal generator of the first implantable stimulation device which is connected to the first processor and produces the signals which are to be sent by a subset of at least two electrodes to the heart of the patient. Alternatively or additionally an ATP therapy or shock therapy triggered by the respective signals produced by the second processor is only allowed to be delivered by the second implantable stimulation device if the above conditions are satisfied. The ATP therapy is provided by a respective signal generator of the second implantable stimulation device which is connected to the second processor and produces signals to be sent by a subset of at least two electrodes connected to the housing of the second implantable stimulation device to the heart of the patient. The shock therapy may be provided by a shock unit of the second implantable stimulation device which is connected to the second processor and produces at least one shock signal via at least one shock coil connected by a lead to the housing of the second implantable stimulation device.

The existence of a pre-defined tachycardia criterion A' or second tachycardia criterion may be revealed if, for example, a pre-defined heart rate or QRS duration is exceeded by the detected cardiac rhythm of the patient is within the time period A or the time period B. Therefore, the first and second processors may be adapted to analyze the detected patient's cardiac rhythm, wherein the detected patient's cardiac rhythm may be realized by a sensed electrocardiogram (ECG). For example, the pre-defined heart rate up to which an ATP-therapy is delivered may be 120 to 180 bpm (beats per minute), in particular 160 bpm. This criterion may be part of the tachycardia criterion A'.

The time period A or the time period B may be regarded as, for example, 10 to 60 seconds, 1, 2, 3 to 5 minutes, in particular 60 seconds in the past from the actual/most recent time point. Time period A and time period B may be different in length, for example the time period A is longer than the time period B.

Moreover, the ATP-therapy delivered by a respective impulse generator of the first implantable stimulation device and/or the second implantable stimulation device may comprise pattern elements, for example, least one burst and/or at least one ramp and/or at least one burst with an extra stimulus, wherein preferably the number of pattern elements is at least 8.

Additionally, the existence or absence of the shock therapy provided by the defibrillator device may be revealed by first processor by the analysis of the detected patient's cardiac rhythm, as well, wherein the detected patient's cardiac rhythm may be given by a sensed ECG. For instance, the detection of an applied shock therapy will be based on the detection of the high voltage applied and optional based on the detection of the typical shock waveform (e.g. biphasic). The decision whether a shock is applied is made by the first processor, for example by a detailed analysis of the ECG signals with regard to the peak height, the peak width and the peak distribution over time. If in the ECG no shock signal is detected within the time period A, the result of the first processor's analysis of the ECG signal is that no shock therapy was provided by the defibrillator device during the time period A.

Further, the existence or absence of the ATP therapy provided by the first implantable stimulation device may be revealed by second processor by the analysis of the detected patient's cardiac rhythm, as well, wherein the detected patient's cardiac rhythm may be given by a sensed ECG. For instance, the detection of a ATP delivery could be based on the recognition of the typical ATP pattern (5.. 10 pacing pulses with a constant interval [Burst] or a decreasing interval length for per inter-pulse interval [Ramp]). The decision whether a shock is applied is made by the second processor, for example by a detailed analysis of the ECG signals with regard to the peak height, the peak width and the peak distribution over time. If in the ECG no ATP therapy signal is detected within the time period B, the result of the second processor's analysis of the ECG signal is that no ATP therapy was provided by the first implantable stimulation device during the time period B.

The above inventive solution shows that the first implantable stimulation device and the second implantable stimulation device of the CRMS use the electrical signals of the respective other device in order to detect whether there was a therapy provided by the other device within a respective pre-defined time period prior the actual/recent time point. Additional communication of the devices, for example using radio waves or other communication channel is not necessary. Accordingly, no additional communication unit/interfaces or energy consumption for separate communication or keeping a communication unit active is necessary. The coordination/management of the therapy of the at least two devices works by using the electrical signals produced by the respective patient or of an implanted device for the therapy of the same patient. Relevant electrical signals are for instance pacing pulses (amplitudes between 0.2 to 10V, pulse width 0.1 to 1,5ms), and defibrillations shocks (monophasic or biphasic waveform; 100 to 1400V; pulse duration 4 to 80ms). Further, the usage of the inventive CRMS is user friendly as well as simple and cost-effective in its structure.

In one embodiment, the first processor is adapted to prevent delivery of signals for ATP therapy for a time period C if the analysis of the patient's cardiac rhythm within the time period A reveals delivery of a shock therapy. In one embodiment, the prevention of the delivery of signals for ATP therapy may be permanently or until an operator re-activates the delivery of signals for ATP therapy. Thus, the time period C may last until a check of the CRMS is performed and, as a result of this, the time period C is terminated by an operator. Additionally, the time period C may be terminated if a pre-defined criterion is detected that reveals the termination of an arrhythmia. Alternatively, the time period C may have a certain, pre-defined length of 3 to 5, 6 to 10, 10 to 15, 15 to 20, 20 to 25, 25 to 30, 30 to 40, 40 to 50, 50 to 60 seconds, or 1 to 1.5, 1.5 to 2, 2 to 2.5, 2.5 to 3, 3 to 5 minutes, 5 to 60 minutes, to multiple hours. If, after revealing of delivery of a shock therapy, the first processor would not be adapted to prevent delivery of signals for ATP-therapy for a time period C, the ATP- and shock therapy could be applied simultaneously or within a close timely proximity. However, a simultaneous application of ATP- and shock therapy may influence the effectiveness of either one of the therapies. More importantly, a simultaneous application of both therapies may even harm the patient as the point in time, in which a shock should be applied to be effective, may be missed as the patient's cardiac rhythm is often changed caused by the application of ATP therapy.

In one embodiment, the second processor is adapted to prevent delivery of signals for shock therapy or a second ATP therapy for a time period D if the analysis of the patient's cardiac rhythm within the time period B reveals delivery of a first ATP therapy. In one embodiment, the prevention of the delivery of signals for shock therapy or the second ATP-therapy may be permanently or until an operator re-activates the delivery of signals for shock therapy or second ATP therapy. Thus, the time period D may last until a check of the CRMS is performed and, as a result of this, the time period D is terminated by an operator. Additionally, the time period D may be terminated if a pre-defined criterion is detected that reveals the termination of an arrhythmia. Alternatively, the time period D may have a certain, pre-defined length of 3 to 5, 6 to 10, 10 to 15, 15 to 20, 20 to 25, 25 to 30, 30 to 40, 40 to 50, 50 to 60 seconds, or 1 to 1.5, 1.5 to 2, 2 to 2.5, 2.5 to 3, 3 to 5 minutes, 5 to 60 minutes, to multiple hours. If, after revealing of delivery of a first ATP therapy, the second processor would not be adapted to prevent delivery of signals for shock therapy or second ATP therapy for a time period D the second ATP therapy or shock therapy may be applied simultaneously with the first ATP therapy or in close timely proximity. As indicated above, a simultaneous application of ATP and shock therapy may influence the effectiveness of either one of the therapies or may harm the patient.

The time period D may last as long as a time period needed for the application of the first ATP therapy. In order to apply shock therapy promptly after ATP therapy, it can be advantageous if the second implantable stimulation device starts loading for the at least one shock during ATP therapy. A more battery-saving variant of this premature start of charging, includes, in particular, a continuous analysis of the detected patient's cardiac rhythm, so that the start of loading for the shock begins when an ongoing arrhythmia following the first ATP therapy seems likely.

In one embodiment, the second processor is adapted to allow delivery of signals for shock therapy if the patient's cardiac rhythm within the time period B reveals a heart rate above a pre-defined limit. In this case, an immediate application of shock therapy may be needed in order to help the patient. Moreover, a shock may be delivered based on the parameters (alone or in combination) of a pathologic QRS waveform, a pathologic ECG signal pattern (e.g. over 10..20 sec), sudden rate increase, and /or rate stability/instability.

In one embodiment the first processor is adapted to re-analyze the patient's cardiac rhythm according to the pre-defined tachycardia criterion A' during the time period C and/or the second processor is adapted to re-analyze the patient's cardiac rhythm according to the pre-defined tachycardia criterion B' during the time period D. This means that if a shock therapy or an ATP therapy occurred a re-analysis of the patient's cardiac rhythm is provided, namely during the time period C or the time period D. In this embodiment, the re-analysis of the patient's cardiac rhythm is provided in the same way as during initial detection of a tachycardia, namely using the predefined tachycardia criterion A' or the predefined tachycardia criterion B'. For instance, time period C or time period D may have a length of 5 to 120 seconds.

In another embodiment during the time period C or the time period B different predefined tachycardia criteria are used for the analysis of the patient's cardiac rhythm. In this embodiment, the first processor is adapted to re-analyze the patient's cardiac rhythm according to a pre-defined tachycardia criterion C' during the time period C and/or the second processor is adapted to re-analyze the patient's cardiac rhythm according to a pre-defined tachycardia criterion D' during the time period D, wherein the pre-defined tachycardia criterion C' is different from the pre-defined tachycardia criterion A' and the pre-defined tachycardia criterion D' is different from the pre-defined tachycardia criterion B'.

The existence of a pre-defined tachycardia criterion C' or tachycardia criterion D' may be revealed if, for example, a pre-defined heart rate or QRS duration is exceeded by the detected cardiac rhythm of the patient is within the time period C or the time period D. A pre-defined heart rate is for example heart rate 120 to 220 bpm. Therefore, the first and second processors may be adapted to analyze the detected patient's cardiac rhythm, wherein the detected patient's cardiac rhythm may be realized by a sensed electrocardiogram (ECG).

In one embodiment the first processor and/or the second processor are/is adapted to terminate the time period C and/or the time period D upon receipt of a termination signal. This termination signal may be provided by an operator or a program routine, for example via a communication to an external device. For example, if the delivery of an ATP therapy is detected during a loading time period of a shock unit for shock delivery a re-evaluation of the patient's cardiac rhythm is provided during a short confirmation detection phase.

As the delivery of at least one shock during shock therapy by the second implantable stimulation device needs a high voltage the shock therapy comprises a loading/charging time period prior the at least one shock is applied by the shock unit to the patient. This loading time period is needed in order to charge at least one capacitor or another capacitive element of the shock unit.

Even if, in one embodiment, the shock therapy is started by loading/charging at least one capacitor of the shock unit, in this embodiment the application of the at least one shock by the shock unit is prevented if the patient's cardiac rhythm within a time period E during or after the loading time period does not correspond to a pre-defined tachycardia criterion E', wherein the pre-defined tachycardia criterion E' may be different from any one or several of the tachycardia criteria A' - D'. Time period E may be for instance 1 to 10 seconds after charging. The charging process typically takes 1 to 24 seconds. The existence of a pre-defined tachycardia criterion E' may be revealed if, for example, a pre-defined heart rate or QRS duration is exceeded by the detected cardiac rhythm of the patient is within the time period E. Therefore, the first and second processors may be adapted to analyze the detected patient's cardiac rhythm, wherein the detected patient's cardiac rhythm may be realized by a sensed electrocardiogram (ECG). Such pre-defined tachycardia criterion E' may comprise the heart rate. If the detected heart rate is below a predefined heart rate, for example, the application of at least one shock is not necessary. This may reduce the stress for the patient. Pre-defined tachycardia criteria E' may be (alone or in combination) a heart rate from 120 to 220 bpm, pathologic QRS waveform, pathologic ECG signal pattern (e.g. over 10 to 20 sec), sudden rate increase, rate stability/instability.

In one embodiment the first processor of the first implantable stimulation device is adapted to deliver signals for at least one other cardiac therapy such as bradycardia pacing therapy or cardiac stimulation for blood pressure reduction.

In stimulation for blood pressure reduction, the timing for stimulating the atrium is performed slightly non-synchronically with respect to a physiologically effective cardiac cycle. As a result, a reduction of the patient's blood pressure is achieved.

The present invention will now be described in further detail with reference to the accompanying schematic drawings, wherein
- Fig. 1: shows an exemplary implantation of an inventive cardiac rhythm management system within a human patient's body,
- Fig. 2: depicts a flow chart of an inventive cardiac rhythm management method,
- Fig. 3 to 5: show the operation of another embodiment of the cardiac rhythm management method during shock therapy,
- Fig. 6: shows an electrocardiogram containing signals of an anti-tachycardia pacing therapy and
- Fig. 7: shows an electrocardiogram containing a signal of a shock therapy.

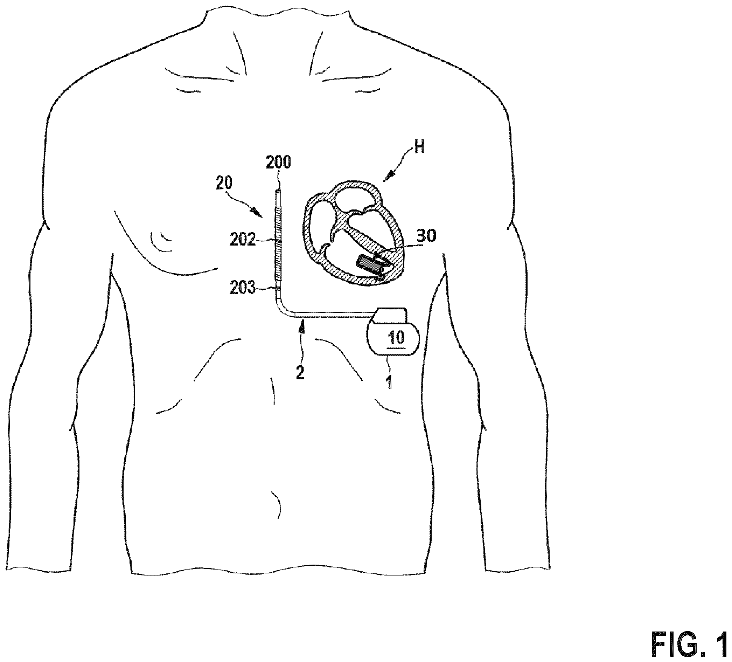

Fig. 1 shows a first embodiment of a cardiac rhythm management system implanted in a human patient's body. The system comprises a subcutaneous implantable cardioverter defibrillator (S-ICD) 1 as a second implantable stimulation device with a housing 10 and an electrode lead 2 connected to the housing 10. Further, the system comprises an ILP (ILP) 30 as a first implantable stimulation device implanted within one, e.g. the right, ventricle of the heart H.

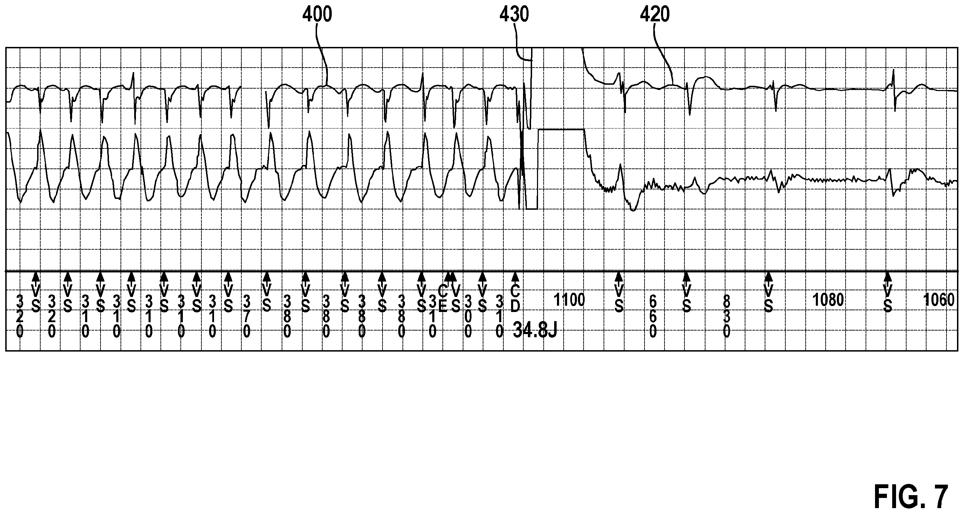

The ILP 30 comprises one or more electrodes as a first detection unit for sensing electric signals of the heart H. Further, the one more electrodes may be used for application anti-tachycardia pacing (ATP) therapy or other cardiac therapy. The detected electric signals are transmitted to a first processor accommodated within the ILP 30. The first processor analyzes the electric signals of the heart (e.g. ECG signals) and thereby determines the patient's cardiac rhythm which may comprise, as shown in Fig. 7, a normal cardiac rhythm 420, a ventricular tachyarrhythmia 400 or a shock 430 provided by the S-ICD as an anti-tachycardia therapy. The first processor is further adapted to generate and deliver signals for ATP therapy to the one or more electrodes of the ILP 30. The functions of the first processor regarding the ATP therapy may be activatable by an operator, for example via a communication link to an external control unit.

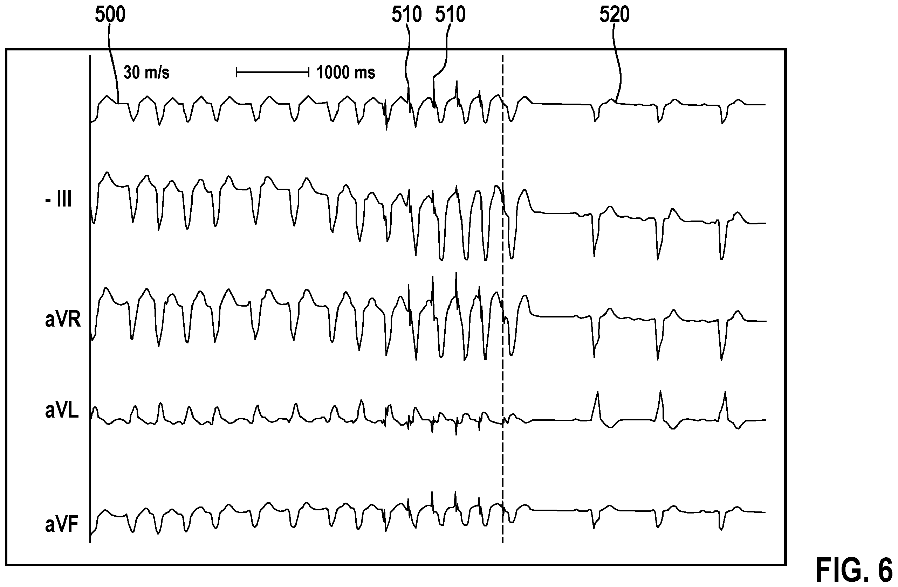

The S-ICD 1 comprises within its housing 10 a second processor and a shock unit. The lead 2 is implanted subcutaneous along the sternum 20 and comprises two detection electrodes 200, 203 as a second detection unit and a shock coil 202 for application of a cardioversion or defibrillation shock. The detection electrodes 200, 203 detect electric signals of and around the heart H and transmit these signals to the second processor accommodated within the housing 10. The second processor analyzes the electric signals of the heart (e.g. the ECG signals) and thereby determines the patient's cardiac rhythm which may comprise, as shown in Fig. 6, a normal cardiac rhythm 520, a tachyarrhythmia 500 or ATP signals 510 provided by the ILP 30 as an anti-tachycardia therapy. The second processor is further adapted to generate and deliver signals transmitted to the shock unit which generates at least one cardioversion or defibrillation shock for anti-tachycardia therapy to shock coil 202. The shock unit has to run through a loading/charging time in which the necessary electric voltage is generated for the one or more shocks delivered by the shock coil 202 as shock therapy.

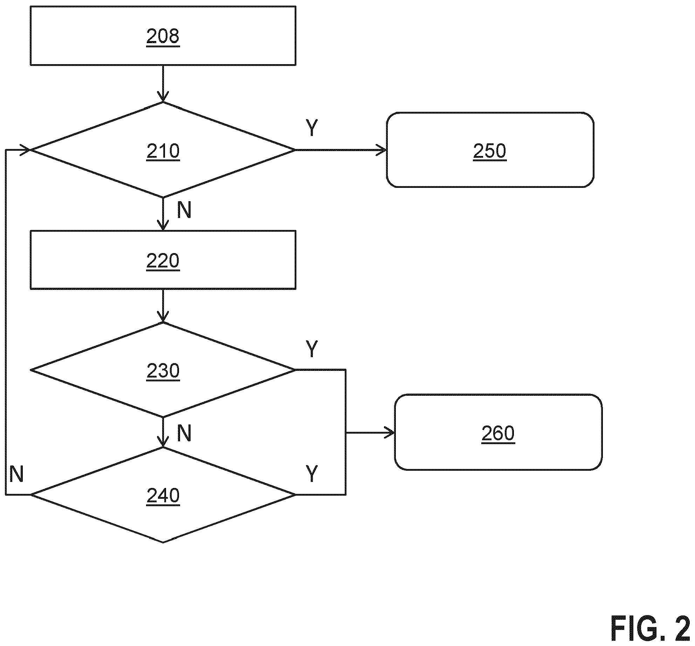

The flow chart of Fig. 2 shows the inventive cardiac rhythm management method with regard using ECG signals as signals showing the patient's cardiac rhythm to the ILP 30. In the first step (see step 208 in Fig. 2) the one more electrodes of the first detection unit of the ILP 30 receive electric signals of the heart H and transmit them to the first processor which analyzes the respective ECG. If the ECG contains a tachycardia the first processor detects this tachycardia initially. In the next step 210 the first processor then checks whether there is any application of shock therapy by a respective shock signal (see signal 430 in Fig. 7) during a preceding time period A. If there is no shock therapy during the time period A, in the next step 220 an ATP therapy may be provided by the ILP 30. Accordingly, the first processor generates signals based on which the one or more electrodes of the ILP 30 apply ATP pulses to the heart H. In the following steps 230 and 240, the first processor further checks whether the tachycardia is terminated and/or the maximum number of ATP therapy units is applied. If none of these criteria is fulfilled, the method continues with step 210 (see above). If at least one of these criteria is fulfilled, the application of any ATP therapy is prevented until the next tachycardia of the heart H is detected by the first processor (see step 260). If there is any shock therapy revealed during the time period A, in the step 250 the ATP therapy is prevented during a time period C. The time period C may continue over a pre-defined time or until the next operator's check of the ILP 30.

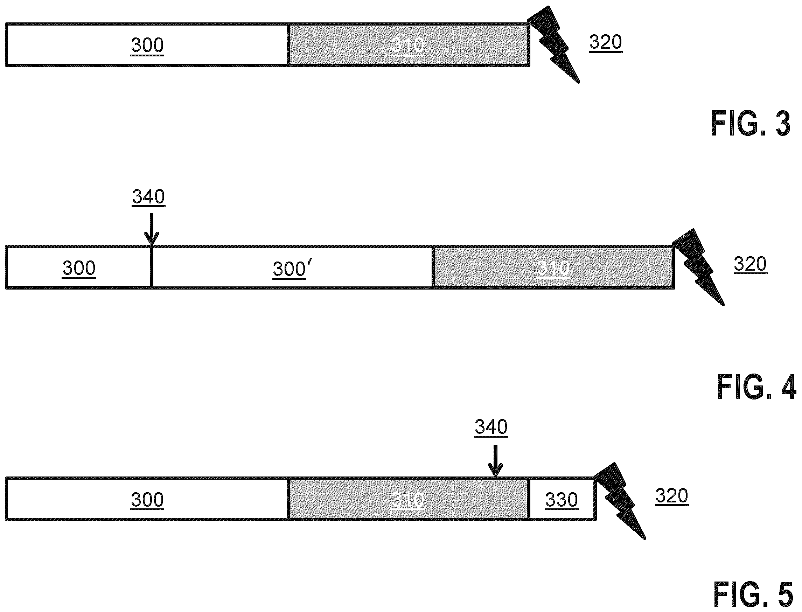

Analog to the flow chart of Fig. 2 the inventive cardiac rhythm management method may be realized also with regard to the S-ICD 1. If the detected ECG signals show a tachycardia the second processor detects this tachycardia initially. In the next step the second processor then checks whether there is any application of ATP therapy by respective ATP signals (see signals 510 in Fig. 6) during a preceding time period B (see time period denoted reference number 300 in Fig. 3). If there is no ATP therapy during the time period B, in the next step a shock therapy may be provided by the S-ICD 1. Accordingly, the second processor generates signals based on which the shock coil 202 of the S-ICD applies one or more shock pulses to the heart H. The shock application (see lighting symbol 320 in Fig. 3) is provided after running through a loading time which is depicted in Fig. 3 by the reference number 310. In the following steps, the second processor further checks whether the Tachycardia is terminated and/or the maximum number of shock therapy units is applied. If none of these criteria is fulfilled, the method continues with checking whether there is any ATP therapy or any tachyarrhythmia during the time period B (see above). If at least one of these criteria is fulfilled, the application of any ATP therapy is prevented until the next tachycardia of the heart H is detected by the second processor. If there is any ATP therapy revealed during the time period B 300, the shock therapy is prevented during a time period D which is in the embodiment shown in Fig. 4 denoted with reference number 300'. The time period D may continue over a pre-defined time period which may be identical to the time period B in length in order to have enough time to check whether after the ATP therapy any tachycardia is still existent, or until the next operator's check of the S-ICD 1.

Further, as indicated in Fig. 5 in one embodiment the delivery of ATP therapy by ILP 30 may be detected during the loading time 310. In this case, after finishing loading time 310 the shock application by the shock unit is prevented during a time period D 330 (confirmation period) in order to check whether after ATP therapy a tachycardia is still detected which needs shock therapy. Only if the tachycardia is still in a dangerous level for the patient, the shock 320 is applied after the time period D 330 has elapsed.

The above system provide a modular cardiac rhythm management of two devices providing anti-tachycardia therapy implanted in the same patient without the necessity of providing any separate communication channel. The system just use the analysis of the patient's cardia rhythm provided anyway for co-ordination of the operation of each device. Accordingly, the system is cost-effective and power-saving as well as reliable and user-friendly. The inventive system avoids cyber-attacks

### Reference numerals

- 1: S-ICD
- 2: electrode lead
- H: heart
- 10: housing
- 20: sternum
- 30: ILP
- 200: detection electrode
- 202: shock coil
- 203: detection electrode
- 208: step of an inventive method for cardiac rhythm management
- 210: step of an inventive method for cardiac rhythm management
- 220: step of an inventive method for cardiac rhythm management
- 230: step of an inventive method for cardiac rhythm management
- 240: step of an inventive method for cardiac rhythm management
- 250: step of an inventive method for cardiac rhythm management
- 260: step of an inventive method for cardiac rhythm management
- 300: time period B in which an initial tachycardia is detected
- 300': time period D during which a shock therapy is prevented for check of tachycardiac properties of the cardiac signal
- 310: loading time
- 320: shock
- 330: time period D during which a shock therapy is prevented for check of tachycardiac properties of the cardiac signal
- 340: time point of detected ATP therapy
- 400: tachyarrhythmia
- 410: ATP therapy
- 420: normal cardiac rhythm
- 430: shock therapy
- 500: tachyarrhythmia
- 510: ATP signal
- 520: normal cardiac rhythm

## Claims

1. A cardiac rhythm management system, comprising:
at least one first implantable stimulation device and at least one second implantable stimulation device,
wherein the at least one first implantable stimulation device comprises a first detection unit adapted to detect a patient's cardiac rhythm and a first processor adapted to analyze the detected patient's cardiac rhythm and to deliver signals for a first antitachycardia pacing therapy,
wherein the at least one second implantable stimulation device comprises a second detection unit (200, 203) adapted to detect the patient's cardiac rhythm and a second processor adapted to analyze the detected patient's cardiac rhythm and to deliver signals for shock therapy or a second antitachycardia pacing therapy, and
wherein the first processor is adapted to allow delivery of signals for antitachycardia pacing therapy only if the analysis of the patient's cardiac rhythm within a pre-defined preceding time period A reveals a pre-defined tachycardia criterion A' and an absence of a shock therapy, and/or
wherein the second processor is adapted to allow delivery of signals for shock therapy or a second antitachycardia therapy only if the analysis of the patient's cardiac rhythm within a pre-defined preceding time period B (300) reveals a pre-defined tachycardia criterion B' and an absence of a first antitachycardia pacing therapy provided by the at least one first implantable stimulation device.

2. The cardiac rhythm management system according to claim 1, wherein the first processor is adapted to prevent delivery of signals for antitachycardia pacing therapy for a time period C if the analysis of the patient's cardiac rhythm within the time period A reveals delivery of a shock therapy.

3. The cardiac rhythm management system according to claim 1 or 2, wherein the second processor is adapted to prevent delivery of signals for shock therapy or a second antitachycardia pacing therapy for a time period D (300', 330) if the analysis of the patient's cardiac rhythm within the time period B reveals delivery of a first antitachycardia pacing therapy.

4. The cardiac rhythm management system according to any of the claims 2 to 3, wherein the first processor is adapted to re-analyze the patient's cardiac rhythm according to the pre-defined tachycardia criterion A' during the time period C and/or the second processor is adapted to re-analyze the patient's cardiac rhythm according to the pre-defined tachycardia criterion B' during the time period D (300', 330).

5. The cardiac rhythm management system according to any of the claims 2 to 3, wherein the first processor is adapted to re-analyze the patient's cardiac rhythm according to a pre-defined tachycardia criterion C' during the time period C and/or the second processor is adapted to re-analyze the patient's cardiac rhythm according to a pre-defined tachycardia criterion D' during the time period D, wherein the pre-defined tachycardia criterion C' is different from the pre-defined tachycardia criterion A' and the pre-defined tachycardia criterion D' is different from the second pre-defined tachycardia criterion.

6. The cardiac rhythm management system according to any of the claims 2 to 5, wherein the first and/or second processor are/is adapted to terminate the time period C and/or the time period D upon receipt of a termination signal.

7. The cardiac rhythm management system according to any of the preceding claims, wherein the delivery of at least one shock during the shock therapy by a shock unit of the second implantable stimulation device comprises a loading time period (310) prior the at least one shock is applied by the shock unit to the patient, wherein the application of the at least one shock by the shock unit is prevented if the patient's cardiac rhythm within a time period E (330) during or after the loading time period (310) does not correspond to a pre-defined tachycardia criterion E'.

8. The cardiac rhythm management system according to any of the preceding claims, wherein the first implantable stimulation device is an implantable leadless pacemaker (II,P), and the second implantable stimulation device is a subcutaneous implantable cardioverter defibrillator (1) or a second ILP.

## Patentansprüche

1. Herzrhythmus-Managementsystem, umfassend:
mindestens eine erste implantierbare Stimulationsvorrichtung und mindestens eine zweite implantierbare Stimulationsvorrichtung,
wobei die mindestens eine erste Stimulationsvorrichtung umfasst: eine erste Detektionseinheit, die dafür ausgelegt ist, einen Herzrhythmus eines Patienten zu detektieren, und einen ersten Prozessor, der dafür ausgelegt ist, den detektierten Herzrhythmus des Patienten zu analysieren und Signale für eine erste Anti tachykarde Stimulationstherapie auszugeben,
wobei die mindestens eine zweite Stimulationsvorrichtung umfasst: eine zweite Detektionseinheit (200, 203), die dafür ausgelegt ist, den Herzrhythmus des Patienten zu detektieren, und einen zweiten Prozessor, der dafür ausgelegt ist, den detektierten Herzrhythmus des Patienten zu analysieren und Signale für eine Schocktherapie oder eine zweite antitachykarde Stimulationstherapie auszugeben, und
wobei der erste Prozessor dafür ausgelegt ist, eine Ausgabe von Signalen für eine antitachykarde Stimulationstherapie nur dann zuzulassen, wenn die Analyse des Herzrhythmus des Patienten innerhalb einer vordefinierten vorausgegangenen Zeitperiode A ein vordefiniertes Tachykardiekriterium A' und eine nicht-stattgefundene Schocktherapie zeigt, und/oder
wobei der zweite Prozessor dafür ausgelegt ist, eine Ausgabe von Signalen für eine Schocktherapie oder eine antitachykarde Stimulationstherapie nur dann zuzulassen, wenn die Analyse des Herzrhythmus des Patienten innerhalb einer vordefinierten vorausgegangenen Zeitperiode B (300) ein vordefiniertes Tachykardiekriterium B' und eine nicht-stattgefundene erste tachykarde Stimulationstherapie, die von der mindestens einen ersten implantierbaren Stimulationsvorrichtung bereitgestellt worden wäre, zeigt.

2. Herzrhythmus-Managementsystem nach Anspruch 1, wobei der erste Prozessor dafür ausgelegt ist, eine Ausgabe von Signalen für eine antitachykarde Stimulationstherapie für eine Zeitperiode C zu verhindern, wenn die Analyse des Herzrhythmus des Patienten innerhalb der Zeitperiode A die Abgabe einer Schocktherapie zeigt.

3. Herzrhythmus-Managementsystem nach Anspruch 1 oder 2, wobei der zweite Prozessor dafür ausgelegt ist, eine Ausgabe von Signalen für eine Schocktherapie oder eine zweite antitachykarde Stimulationstherapie für eine Zeitperiode D (300', 330) zu verhindern, wenn die Analyse des Herzrhythmus des Patienten innerhalb der Zeitperiode B die Abgabe einer ersten tachykarden Stimulationstherapie zeigt.

4. Herzrhythmus-Managementsystem nach einem der Ansprüche 2 bis 3, wobei der erste Prozessor dafür ausgelegt ist, während der Zeitperiode C den Herzrhythmus des Patienten gemäß dem vordefinierten Tachykardiekriterium A' neu zu analysieren, und/oder der zweite Prozessor dafür ausgelegt ist, den Herzrhythmus des Patienten während der Zeitperiode D (300', 330) gemäß dem vordefinierten Tachykardiekriterium B' neu zu analysieren.

5. Herzrhythmus-Managementsystem nach einem der Ansprüche 2 bis 3, wobei der erste Prozessor dafür ausgelegt ist, den Herzrhythmus des Patienten während der Zeitperiode C gemäß einem vordefinierten Tachykardiekriterium C' neu zu analysieren, und/oder der zweite Prozessor dafür ausgelegt ist, den Herzrhythmus des Patienten während der Zeitperiode D gemäß einem vordefinierten Tachykardiekriterium D' neu zu analysieren, wobei das vordefinierte Tachykardiekriterium C' von dem vordefinierten Tachykardiekriterium A' verschieden ist und das vordefinierte Tachykardiekriterium D' von dem zweiten vordefinierten Tachykardiekriterium verschieden ist.

6. Herzrhythmus-Managementsystem nach einem der Ansprüche 2 bis 5, wobei der erste und/oder der zweite Prozessor dafür ausgelegt ist/sind, die Zeitperiode C und/oder die Zeitperiode D bei Empfang eines Beendigungssignals zu beenden.

7. Herzrhythmus-Managementsystem nach einem der vorangehenden Ansprüche, wobei die Abgabe mindestens eines Schocks durch eine Schockeinheit der zweiten implantierbaren Stimulationsvorrichtung während der Schocktherapie eine Ladezeitperiode (310) umfasst, bevor der mindestens eine Schock von der Schockeinheit an den Patienten abgegeben wird, wobei die Abgabe des mindestens einen Schocks durch die Schockeinheit verhindert wird, wenn der Herzrhythmus des Patienten innerhalb einer Zeitperiode E (330) während oder nach der Ladezeitperiode (310) einem vordefinierten Tachykardiekriterium E' nicht entspricht.

8. Herzrhythmus-Managementsystem nach einem der vorangehenden Ansprüche, wobei die erste implantierbare Stimulationsvorrichtung ein implantierbarer drahtloser Schrittmacher (ILP) ist und die zweite implantierbare Stimulationsvorrichtung ein subkutaner implantierbarer Kardioverter-Defibrillator (1) oder ein zweiter ILP ist.

## Revendications

1. Système de gestion de rythme cardiaque, comprenant :
au moins un premier dispositif de stimulation implantable et au moins un second dispositif de stimulation implantable,
dans lequel l'au moins un premier dispositif de stimulation implantable comprend une première unité de détection adaptée pour détecter le rythme cardiaque d'un patient et un premier processeur adapté pour analyser le rythme cardiaque détecté du patient et pour administrer des signaux pour une première thérapie de stimulation antitachycardie,
dans lequel l'au moins un second dispositif de stimulation implantable comprend une seconde unité de détection (200, 203) adaptée pour détecter le rythme cardiaque du patient et un second processeur adapté pour analyser le rythme cardiaque détecté du patient et pour administrer des signaux pour une thérapie de choc ou une seconde thérapie de stimulation antitachycardie, et
dans lequel le premier processeur est adapté pour permettre l'administration de signaux pour une thérapie de stimulation antitachycardie uniquement si l'analyse du rythme cardiaque du patient au sein d'une période précédente prédéfinie A révèle un critère de tachycardie prédéfini A' et une absence de thérapie de choc, et/ou
dans lequel le second processeur est adapté pour permettre l'administration de signaux pour une thérapie de choc ou une seconde thérapie antitachycardie uniquement si l'analyse du rythme cardiaque du patient au sein d'une période précédente prédéfinie B (300) révèle un critère de tachycardie prédéfini B' ou une absence d'une première thérapie de stimulation antitachycardie fournie par l'au moins un premier dispositif de stimulation implantable.

2. Système de gestion de rythme cardiaque selon la revendication 1, dans lequel le premier processeur est adapté pour empêcher l'administration de signaux pour une thérapie de stimulation antitachycardie pour une période C si l'analyse du rythme cardiaque du patient au sein de la période A révèle l'administration d'une thérapie de choc.

3. Système de gestion de rythme cardiaque selon la revendication 1 ou 2, dans lequel le second processeur est adapté pour empêcher l'administration de signaux pour une thérapie de choc ou une seconde thérapie de stimulation antitachycardie pour une période D (300', 330) si l'analyse du rythme cardiaque du patient au sein de la période B révèle l'administration d'une première thérapie de stimulation antitachycardie.

4. Système de gestion de rythme cardiaque selon l'une quelconque des revendications 2 à 3, dans lequel le premier processeur est adapté pour ré-analyser le rythme cardiaque du patient selon le critère de tachycardie prédéfini A' pendant la période C et/ou le second processeur est adapté pour ré-analyser le rythme cardiaque du patient selon le critère de tachycardie prédéfini B' pendant la période D (300', 330).

5. Système de gestion de rythme cardiaque selon l'une quelconque des revendications 2 à 3, dans lequel le premier processeur est adapté pour ré-analyser le rythme cardiaque du patient selon un critère de tachycardie prédéfini C' pendant la période C et/ou le second processeur est adapté pour ré-analyser le rythme cardiaque du patient selon un critère de tachycardie prédéfini D' pendant la période D, dans lequel le critère de tachycardie prédéfini C' est différent du critère de tachycardie prédéfini A' et le critère de tachycardie prédéfini D' est différent du second critère de tachycardie prédéfini.

6. Système de gestion de rythme cardiaque selon l'une quelconque des revendications 2 à 5, dans lequel le premier et/ou le second processeur sont adaptés pour mettre fin à la période C et/ou à la période D à la réception d'un signal de fin.

7. Système de gestion de rythme cardiaque selon l'une quelconque des revendications précédentes, dans lequel l'administration d'au moins un choc pendant la thérapie de choc par une unité de choc du second dispositif de stimulation implantable comprend une période de temps de chargement (310) avant que l'au moins un choc ne soit appliqué par l'unité de choc au patient, dans lequel l'application de l'au moins un choc par l'unité de choc est empêché si le rythme cardiaque du patient au sein d'une période E (330) pendant ou après la période de chargement (310) ne correspond pas à un critère de tachycardie prédéfini E'.

8. Système de gestion de rythme cardiaque selon l'une quelconque des revendications précédentes, dans lequel le premier dispositif de stimulation implantable est un stimulateur cardiaque sans câbles implantable (ILP), et le second dispositif de stimulation implantable est un défibrillateur automatique implantable sous-cutané (1) ou un second ILP.
